# EUROPEAN PATENT SPECIFICATION

(11) **EP 3 684 373 B1**
(45) Date of publication and mention of the grant of the patent: **18.11.2026**
(21) Application number: 18858223.3
(22) Date of filing: 20.09.2018
(51) Int. Cl.: A61K 31/7048, A61K 9/06, A61K 9/00, A61K 9/107, A61K 9/127, A61K 9/51, A61P 11/02, A61P 11/06, A61P 11/12, A61P 31/16, A61P 11/08, A61K 45/06, A61K 31/43, A61K 31/438, A61K 31/496, A61K 31/65, A61K 31/7052, A61K 9/1274

(54) **COMPOSITIONS COMPRISING AMPHOTERICIN B, AZITROMYCIN AND RIFABUTIN FOR TREATING AND AMELIORATING RESPIRATORY CONDITIONS AND INFLAMMATION OF MUCOSA**
ZUSAMMENSETZUNGEN AUS AMPHOTERICIN B, AZITROMYCIN UND RIFABUTIN ZUR BEHANDLUNG UND LINDERUNG VON ATEMWEGSERKRANKUNGEN UND SCHLEIMHAUTENTZÜNDUNGEN
COMPOSITIONS COMPRENANT DE L'AMPHOTÉRICINE B, DE L'AZITROMYCINE ET DE LA RIFABUTINE POUR LE TRAITEMENT ET L'AMÉLIORATION DES AFFECTIONS RESPIRATOIRES ET DE L'INFLAMMATION DES MUQUEUSES

(30) Priority: 20.09.2017 US 201762561146 P; 21.09.2017 US 201762561636 P
(43) Date of publication of application: 29.07.2020
(73) Proprietor: Atopic Medical, Inc., Lincoln, MA 01773 (US)
(72) Inventor: BORODY, Thomas, Julius, Five Dock NSW 2046 (AU); PEATTIE, Debra, Lincoln MA 01773 (US)
(74) Representative: HGF
(86) International application number: PCT/US2018/051965
(87) International publication number: WO 2019/060553

(56) References cited:
- WO-A1-03/061767
- WO-A1-2004/091576
- WO-A1-2010/091090
- WO-A1-2012/151517
- WO-A1-2014/022414
- WO-A1-97/36479
- US-A1- 2003 219 473
- US-A1- 2005 159 369
- US-A1- 2006 025 355
- US-A1- 2014 371 134
- US-A1- 2015 291 648

## Description

### BACKGROUND

Inflammation and/or infection of the mucosa of the respiratory tract, sometimes called "mucositis", is a common finding in the respiratory tract, particularly in symptomatic patients. It is generally caused by one or more chronic infections. In the respiratory tract the infection and/or the inflammation affects the nose, sinuses, large airways, and smaller airways such as bronchi and bronchioles, as well as the lung tissues at times. This inflammation carries various names including rhinosinusitis, sinusitis, tracheo-bronchitis, bronchiolitis, and at a several levels the inflammation can result in a clinical condition called "asthma".

Asthma is a common disorder that causes much morbidity and significant mortality. Asthma affects at least 25 million people in the US, with over a half a million patients being admitted to hospital at least yearly due to severe asthma attacks.

The general cause of this chronic infection and/or inflammation, of the respiratory tract, remains generally unknown; however, a number of superficial and at times co-existing infections can be found, which include numerous fungal organisms such as *Chlamydophila pneumonia(Cpn), Haemophilus influenzae,* and *Mycoplasma pneumoniae.*

Various therapies are used to improve air movement between the mouth and the lungs, requiring at times steroid therapy, adrenalin usage, anti-inflammatory agents, and various other methodologies.

Current therapies fail to address infective components in the mucosa contributing to mucositis. Rather, the abovementioned treatments tend to address secondary, inflammatory processes that accompany such infections. This approach to treatment is similar to that used in Crohn's disease and ulcerative colitis, where steroids and other immunomodulators such as azathioprine or infliximab are used instead of targeting underlying infective causes.

In US Patent 6,291,500, Ponikau described the use of mucosal or inhaled administration of anti-fungal agents. He described the treatment of non-invasive fungus-induced mucositis by administration of a formulation of anti-fungal agents. Vyden, in WO 02/07682, described the treatment of atopic disorders including asthma using an anti-fungal agent such as Lamisil (terbinafine) together with an antibiotic, where the treatment also required reduction or cessation of use of emollients by the patient, to try and minimize fungal spore inhalation.

US 7,241,741 used an antifungal agent combined with two antimicrobial agents. These medications were ingested to treat not only a superficial mucositis but rather to treat deeper, penetrating mucosal infection by using an antifungal agent and antibiotics. The antifungal of choice in this patent was terbinafine hydrochloride. Later, itraconazole hydrochloride was found to be more effective; see also US 7,776,850.

With the passage of time and growing clinical experience, however, the terbinafine hydrochloride and hydrochloride antifungal agents were found to have clinical disadvantages, as seen, for example, as a cross-reaction with other drugs such as itraconazole hydrochloride, rifabutin and/or clarithromycin or as development of liver damage upon administration of terbinafine hydrochloride.

Amphotericin B (AMB) is an antifungal used as an injectable or inhaled agent because of its inability to be absorbed when ingested; also, it has significant renal toxicity when given parenterally, see USPN 7,241,741. Some reports of inhaled parenteral AMB in asthma have appeared, but responses have been quite variable.

Other antifungal formulations have been attempted. There has been some effort to formulate AMB for oral administration. These include formulating AMB as nanosuspensions (Kayser et al., 2003), as Poly(lactide-co-glycolide) nanoparticles (NPs) employing vitamin E-TPGS as a stabilizer (Italia et al., 2009; Italia et al., 2011), as lipid-based oral formulation using Peceol (Sachs-Barrable et al., 2008) or as liquid antisolvent precipitation NPs (Zu et al., 2014). Furthermore, AMB has been loaded to Peceol and PEG-phospholipids (iCo-009) (Gershkovich et al., 2010; Sivak et al., 2011), to carbon nanotubes (Prajapati et al., 2012), to gelatin-coated lipid NPs (Jain et al., 2012), to Chitosan-EDTA conjugates (Singh et al., 2013) and to Cubosomes (Yang et al., 2012; Yang et al., 2014), polymeric nanoparticles (Verma RK, Pandya S, Misra A. Loading and release of amphotericin-B from biodegradable poly(lactic-co-glycolic acid) nanoparticles. J Biomed Nanotechnol. 2011;7(1):118-120, nanosuspensions (Golenser J, Domb A. New formulations and derivatives of amphotericin B for treatment of leishmaniasis. Mini Rev Med Chem. 2006;6(2):153-162, solid lipid nanoparticles (Patel PA, Patravale VB. AmbiOnp: solid lipid nanoparticles of amphotericin B for oral administration. J Biomed Nanotechnol. 2011;7(5):632-639. The most recent reports include AMB liposomes containing ceramides (Skiba-Lahiani et al., 2015) and AMB encapsulated with a chitosan derivative (Serrano et al., 2015). These oral drug deliveries were developed to enhance the solubility and gastrointestinal permeability of AMB. In most cases, these formulations failed to increase absorption of orally administered AMB, and none of them has been introduced to the market (Ibrahim et al., 2012; Yang et al., 2012). Radwan et al. (2017) developed a novel PEGylated polylactic-polyglycolic acid copolymer (PLGA-PEG) nanoparticle (NP) formulations of AMB with high ability to kill *Candida albicans.*
There was little nephrotoxicity in rats, and the addition of glycyrrhizic acid (GA) to AMB NPs formulation resulted in a significantly increased oral absorption and improved bioavailability in rats. WO03/061767 discloses methods for the treatment of respiratory tract mucositis in individuals in need of such treatment, the method comprising the systemic administration to the individual of therapeutically effective amounts of at least one anti-fungal agent and at least one anti-bacterial agent, wherein the treatment does not involve the cessation of use of emollients by the individual. WO2004/091576 discloses methods and formulations for treating or preventing rhinosinusitis, including fungus-induced rhinosinusitis in mammals; the formulation may comprise a steroidal anti-inflammatory agent having a specific particle size distribution profile; and the formulation may also comprise an antifungal agent, antibiotic or antiviral agent. US 2014/0371134 discloses a dry formulation for the effective administration of multiple medications simultaneously for one or more ailments. The dry formulations include one or more of the following actives in combination with pharmaceutically acceptable excipients or additives: a) at least one antibiotic; b) at least one anti-inflammatory steroid; and c) at least one antifungal agent.

### SUMMARY

The invention is defined in the appended claims. In an aspect, the present invention relates to a therapeutic combination of drugs comprising Amphotericin B, azithromycin, and rifabutin, wherein the Amphotericin B is formulated for administration by inhalation wherein the amphotericin B is formulated as a spray, an aerosol or a powder. The invention also provides the therapeutic combination of drugs for use in the treatment of an asthma, a bronchitis, a sinusitis, or rhinosinusitis. The references to methods of treatment of the human or animal body by therapy or surgery, or diagnostic methods practised on the human or animal body, are to be interpreted as references to the compounds, compositions or products of the invention for use in such methods. In alternative embodiments, the therapeutic combinations may be for use in methods for treating, ameliorating, reversing and/or preventing (acting as a prophylaxis): a respiratory condition involving an infection or an inflammation, or any lung condition involving inflammation or infection, e.g., of a respiratory mucosa, and/or an infection or an inflammation of an underlying muscle of the respiratory tract; or, an asthma; a bronchitis; a sinusitis or rhinosinusitis; an infection of a sinus; chronic obstructive airway disease; emphysema; chronic bronchitis; pneumonia; or, a bronchiectasis. In alternative embodiments, the therapeutic combination comprises a combination of Amphotericin B and: two antibiotics; three antibiotics; or, four or more antibiotics; wherein the therapeutic combination comprises azithromycin, and rifabutin. In alternative embodiments, therapeutic combination of drugs may be dosaged and administered to patients, such as children, in need thereof. In alternative embodiments, compositions and methods provided herein are dosaged and formulated as tablet, capsule, liquid, powder or aerosol preparations or formulations, or preparations or formulations for oral delivery or inhalation.

In alternative embodiments, provided are therapeutic combinations comprising inhalation formulated Amphotericin B,
wherein the therapeutic combination comprises a combination of at least one antifungal agent or composition comprising an Amphotericin Band:
   (b) two antibiotics or antibacterial agents;
   (c) three antibiotics or antibacterial agents; or
   (d) four or more antibiotics or antibacterial agents,
wherein the therapeutic combination comprises azithromycin, and rifabutin, wherein: the Amphotericin B, is formulated for administration by inhalation,
wherein optionally the formulation for administration by inhalation or intraoral or sublingual administration comprises formulation as a spray, an aerosol or a powder.

According to the invention, the Amphotericin B is formulated for administration by inhalation. In alternative embodiments, the at least one antifungal agent or composition or equivalent antifungal agent or composition formulated for oral administration is formulated for delivery in a capsule, a tablet, a geltab or equivalent, and optionally the at least one antifungal agent or composition or equivalent antifungal agent or composition is formulated for oral administration in the same formulation (optionally the capsule, the tablet, the geltab or equivalent) as the first, second, third and/or fourth or more antibiotic or antibacterial agents.

According to the invention, the Amphotericin B is formulated for administration by inhalation. In alternative embodiments, the at least one antifungal agent or composition or equivalent antifungal agent or composition is packaged for oral administration in a blister pack, a bubble pack, a slide blister package, a tray, a clamshell or a shrink wrap,
and optionally the at least one antifungal agent or composition or equivalent antifungal agent or composition is packaged for oral administration in the same blister pack, bubble pack, slide blister package, tray, clamshell or shrink wrap as the first, second, third and/or fourth or more antibiotic or antibacterial agents,
and optionally the at least one antifungal agent or composition or equivalent antifungal agent or composition is packaged for oral administration in the same blister pack, bubble pack, slide blister package, tray, clamshell or shrink wrap as the first, second, third and/or fourth or more antibiotic or antibacterial agents such that:
   - the at least one antifungal agent or composition or equivalent antifungal agent or composition and the first, second, third and/or fourth or more antibiotic or antibacterial agents are administered or taken together;
   - the at least one antifungal agent or composition or equivalent antifungal agent or composition is administered before the first, second, third and/or fourth or more antibiotic or antibacterial agents; or
   - the at least one antifungal agent or composition or equivalent antifungal agent or composition is administered after the first, second, third and/or fourth or more antibiotic or antibacterial agents,
   - the at least one antifungal agent or composition or equivalent antifungal agent or composition and the first, second, third and/or fourth or more antibiotic or antibacterial agents are administered in pulsed dosages, optionally, comprising starting at a high dose, then a low dose, then a high dose, then a low dose.

In alternative embodiments, the at least one additional anti-fungal agent or composition (in addition to Amphotericin B) is selected from the group consisting of: flucytosine, ketoconazole, miconazole, itraconazole, fluconazole, griseofulvin, clotrimazole, econazole, terconazole, butoconazole, oxiconazole, sulconazole, supraconazole, voriconazole, posaconazole, ciclopirox olamine, haloprogin, tolnaftate, naftifine, terbinafine hydrochloride, a morpholine, nystatin, natamycin, butenafine, undecylenic acid, proprionic acid, caprylic acid and a combination thereof.

In alternative embodiments, the (a) one antibiotic or antibacterial agent; (b) two antibiotics or antibacterial agents; (c) three antibiotics or antibacterial agents; or (d) four or more antibiotics or antibacterial agents (e.g., to be administered with Amphotericin B), comprises: an antibiotic or antibacterial agent used to treat an *Chlamydia pneumoniae* infection, a *Haemophilus influenzae* infection, or a *Mycoplasma pneumoniae* infection.

According to the invention, the therapeutic combination comprises Amphotericin B, azithromycin and rifabutin. In alternative embodiments, (b) two antibiotics or antibacterial agents; (c) three antibiotics or antibacterial agents; or (d) four or more antibiotics or antibacterial agents (e.g., to be administered with Amphotericin B), comprises: an antibiotic or antibacterial agent from one or more of the following classes selected from: tetracyclines, penicillins, macrolides, quinolones, chloramphenicol, rifamycins, sulphonamides, co-trimoxazole, and oxazolidinones.

According to the invention, the therapeutic combination comprises Amphotericin B, azithromycin and rifabutin. In alternative embodiments, (b) two antibiotics or antibacterial agents; (c) three antibiotics or antibacterial agents; or (d) four or more antibiotics or antibacterial agents (e.g., to be administered with Amphotericin B), comprise: a doxycycline, chlortetracycline, tetracycline hydrochloride, oxytetracycline, demeclocycline, methacycline, minocycline, penicillin, amoxycillin, erythromycin, clarithromycin, roxithromycin, azithromycin, spiramycin, oleandomycin, josamycin, kitsamysin, flurithromycin, nalidixic acid, oxolinic acid, norfloxacin, perfloxacin, amifloxacin, ofloxacin, ciprofloxacin, sparfloxacin, levofloxacin, rifabutin, rifampicin, rifapentin, rifalazil, sulfisoxazole, sulfamethoxazole, sulfadiazine, sulfadoxine, sulfasalazine, sulfaphenazole, dapsone, sulfacytidine, linezolid, aminoglycosides (such as Amikacin (Amikin), Gentamicin (Garamycin), Kanamycin (Kantrex), Neomycin (Neo-Fradin), Netilmicin (Netromycin), Tobramycin (Nebcin), Paromomycin (Humatin), Streptomycin (N/A), Spectinomycin (Trobicin)), ansamycins (such as Geldanamycin (Trastuzumab), Herbimycin (N/A), Rifaximin (Xifaxan),Rifabutin(Mycobutin), Rifampicin (Rifampin), Rifalazil, Rifapentine; Tanespimycin), carbacephem (such as Loracarbef (Lorabid)), carbapenems (such as Ertapenem (Invanz), Doripenem (Doribax), Imipenem/Cilastatin (Primaxin), Meropenem (Merrem)), first generation cephalosporins (such as Cefadroxil (Duricef), Cefazolin (Ancef), Cefalexin (Keflex)), second generation cephalosporins (such as Cefaclor (Distaclor), Cefprozil (Cefzil), Cefuroxime (Ceftin, Zinnat)), third generation cephalosporins (such as Cefixime (Cefspan), Cefdinir (Omnicef, Cefdiel), Cefditoren (Spectracef, Meiact), Cefoperazone (Cefobid), Cefotaxime (Claforan), Cefpodoxime (Vantin, Banadoz), Ceftazidime (Fortaz), Ceftibuten (Cedax), Ceftriaxone (Rocephin)), fourth generation cephalosporins (such as Cefepime (Maxipime)), fifth generation cephalosporins (such as Ceftaroline fosamil (Teflaro), Ceftobiprole (Zeftera)), glycopeptides (such as Teicoplanin (Targocid), Vancomycin (Vancocin),Telavancin (Vibativ), Dalbavancin (Dalvance), Oritavancin (Orbactiv)), lincosamides (such as Clindamycin (e.g., CLEOCIN^{™}, DALACIN^{™}, CLINACIN^{™}), Lincomycin (Lincocin)), lipopetide (such as daptomycin (cubicin)), macrolides (such as Azithromycin (Zithromax, Sumamed, Xithrone), Clarithromycin (Biaxin), Erythromycin (Erythocin, Erythroped), Roxithromycin (N/A), Telithromycin (Ketek), Spiramycin (Rovamycine)), monobactams (such as aztreonam (azactam)), nitrofurans (such as Furazolidone (Furoxone), Nitrofurantoin (Macrodantin, Macrobid)), nitroimidazoles (such as Tinidazole (Fasigyn, Simplotan, Tindamax), Metronidazole (Flagyl), Ornidazole (Ornigil), Secnidazole), oxazolidinones (such as Linezolid (Zyvox), Posizolid (N/A), Radezolid (N/A), Torezolid (Sivextro); Cadazolid), penicillins (such as Amoxicillin (Novamox, Amoxil), Ampicillin (Principen), Azlocillin, Dicloxacillin (Dynapen), Flucloxacillin (Floxapen), Mezlocillin (Mezlin), Methicillin (Staphcillin), Nafcillin (Unipen), Oxacillin (Prostaphlin), Penicillin G (Pentids), Penicillin V (Veetids), Piperacillin (Pipracil), Penicillin G (Pfizerpen), Temocillin (Negaban), Ticarcillin (Ticar)), penicillin combinations (such as Amoxicillin/clavulanate (Augmentin), Ampicillin/sulbactam (Unasyn), Piperacillin/tazobactam (Zosyn), Ticarcillin/clavulanate (Timentin)), polypeptides (such as Bacitracin (Baciguent), Colistin (Coly-Mycin-S), Polymyxin B), quinolones/fluoroquinolones (such as Ciprofloxacin (Cipro, Ciproxin, Ciprobay), Enoxacin (Penetrex), Gatifloxacin (Tequin), Gemifloxacin (Factive), Levofloxacin (Levaquin), Lomefloxacin (Maxaquin), Moxifloxacin (Avelox), Nadifloxacin (Nadoxin), Nalidixic acid (NegGram), Norfloxacin (Noroxin), Ofloxacin (Floxin, Ocuflox), Trovafloxacin (Trovan), Grepafloxacin (Raxar), Sparfloxacin (Zagam), Temafloxacin (Omniflox)), sulfonamides (such as Mafenide (Sulfamylon), Sulfacetamide (Sulamyd, Bleph-10), Sulfadiazine (Micro-Sulfon), Silver sulfadiazine (Silvadene), Sulfadimethoxine (Di-Methox, Albon), Sulfamethizole (Thiosulfil Forte), Sulfamethoxazole (Gantanol), Sulfanilimide (N/A), Sulfasalazine (Azulfidine), Sulfisoxazole (Gantrisin), Trimethoprim (Bactrim, Septra), Sulfamethoxazole (Gantanol), Sulfonamidochrysoidine (Prontosil)), tetracyclines (such as Demeclocycline (Declomycin), Doxycycline (Vibramycin), Metacycline, Minocycline (Minocin), Oxytetracycline (Terramycin), Tetracycline (Sumycin, Achromycin V, Steclin)), drugs against mycobacteria (such as Clofazimine (Lamprene), Dapsone (Avlosulfon), Capreomycin (Capastat), Cycloserine (Seromycin), Ethambutol (Myambutol), Ethionamide (Trecator), Isoniazid (Nydrazid), Pyrazinamide (Aldinamide), Rifampicin (Rifadin, Rimactane), Rifabutin (Mycobutin), Rifapentine (Priftin), Streptomycin), Arsphenamine (Salvarsan), Chloramphenicol (Chloromycetin), Fosfomycin (Monurol, Monuril), Fusidic acid, Metronidazole (Flagyl), Mupirocin (Bactroban), Platensimycin, Quinupristin/Dalfopristin (Synercid), Thiamphenicol, Tigecycline (Tigacyl), Tinidazole (Tindamax Fasigyn), Trimethoprim (Proloprim, Trimpex) ; Fidaxomicin (Marocyclic antibiotic - Dificid); Ridinilazole; Ramoplanin; Nitazoxanide; Tizoxanide; Surotomycin, or any combination thereof, wherein the therapeutic combination comprises azithromycin, and rifabutin.

In alternative embodiments, the (b) two antibiotics or antibacterial agents; (c) three antibiotics or antibacterial agents; or (d) four or more antibiotics or antibacterial agents (e.g., to be administered with Amphotericin B), comprises:
(i) a doxycycline and a rifabutin;
(ii) a clarithromycin and a rifampicin;
(iii) a doxycycline and a rifampicin;
(iv) a clarithromycin and a rifabutin;
(v) a tetracycline hydrochloride and a rifampicin;
(vi) a clarithromycin and a rifabutin;
(vii) an azithromycin and a rifampicin;
(viii) an azithromycin and a rifabutin;
(ix) an erythromycin and an amoxycillin;
(x) a clarithromycin and a doxycycline;
(xi) a rifabutin and an azithromycin; or
(xii) any combination thereof; wherein the therapeutic combination comprises azithromycin, and rifabutin.

In variants of the disclosure not forming part of the invention, the Amphotericin B is formulated for oral administration as or in: a nano-suspension delivery system; an encochleated formulation; or, as a multilayer crystalline, spiral structure with no internal aqueous space, wherein optionally the encochleated formulation comprises a lipid-crystal encochleated drug formulation made up of nano-sized particles, wherein optionally the nano-sized particles are about 10 to 1000 nanometers in diameter, or about 20 to 500 nanometers in diameter, or about 50 to 100 nanometers in diameter.

According to the invention, the therapeutic combination comprises, consists essentially of, or consists of amphotericin B, rifabutin, and azithromycin.

In alternative embodiments, provided are therapeutic combination of drugs for use in methods for the treatment of at least one fungal infection, or for the treatment of a co-infection by at least one fungi and at least one other infectious agent, in an individual in need thereof,
wherein optionally the at least one other infectious agent comprises a bacterium,
the method comprising administration to the individual in need thereof a therapeutically effective amount of: therapeutic combination drugs comprising Amphotericin B, azithromycin, and rifabutin, wherein the Amphotericin B is formulated for administration by inhalation wherein the amphotericin B is formulated as a spray, an aerosol or a powder.

In alternative embodiments of therapeutic combination of drugs for use in the methods, the treatment does not involve the cessation or reduction of use of emollients by the individual in need thereof, or the method comprises a caveat that the treatment does not comprise the cessation or reduction of use of emollients in the individual in need thereof.

In alternative embodiments of therapeutic combination of drugs for use in the methods, the treatment comprises cessation or reduction of use of emollients by the individual in need thereof.

In alternative embodiments, therapeutic combination of drugs for use in the method comprises treating, amelioriating, reversing and/or preventing (acting as a prophylaxis):
- a respiratory or a lung condition involving an infection and/or an inflammation, optionally of a respiratory mucosa, and/or an infection or an inflammation of an underlying muscle of the respiratory tract,
   wherein optionally the underlying muscle of the respiratory tract is a smooth muscle, or a bronchial or a bronchiole smooth muscle,
   wherein optionally the inflammation is a chronic or an acute inflammation, wherein optionally the inflammation is secondary or related to cystic fibrosis, or is a cystic fibrosis-associated lung disease,
   wherein optionally the sinus is a paranasal sinus,
   and optionally the infection is caused at least in part by a fungus, wherein optionally the fungus comprises an *Aspergillus* species, or an *Aspergillus fumigatus,* an *Aspergillus flavus* or an *Aspergillus niger,* or *Scedosporium, Fusarium, Paecilomyces, Acremonium, Trichoderma, Cryptococcus gatti,* or *Histoplasma capsulatum, Coccidioides immitis, Blastomyces dermatitidis, Paracoccidioides brasiliensis, Sporothrix schenckii, Cryptococcus neoformans, Candida* species, *Mucor* species, *Pneumocystis* including *jiroveci, Blastomycosis, Zygomycosis, Bipolaris* species, *Schizophyllum commune, Curvularia* species, *Pseudallescheria boydii* species complex, *Alternaria alternata, Fusarium vasinfectum, Penicillium* species, *Cladosporium cladosporioides, Stemphylium languinosum, Rhizopus oryzae, Candida glabrata, Saccharomyces cerevisiae, Schizophyllum commune,* and *Trichosporon beigelii,*
   and optionally the infection is or causes a pneumonia,
- an asthma,
- a bronchitis, optionally, a chronic bronchitis,
- a sinusitis or rhinosinusitis, or infection of a sinus,
   wherein optionally the sinus is a paranasal sinus, and optionally the infection is caused at least in part by a fungus, wherein optionally the fungus comprises an *Aspergillus* species, or an *Aspergillus fumigatus,* an *Aspergillus flavus* or an *Aspergillus niger,* or *Scedosporium, Fusarium, Paecilomyces, Acremonium, Trichoderma and Cryptococcus gatti,* or *Histoplasma capsulatum, Coccidioides immitis, Blastomyces dermatitidis, Paracoccidioides brasiliensis, Sporothrix schenckii, Cryptococcus neoformans, Candida* species, *Mucor* species, *Pneumocystis* including *jiroveci, Blastomycosis, Zygomycosis, Bipolaris* species, *Schizophyllum commune, Curvularia* species, *Pseudallescheria boydii* species complex, *Alternaria alternata, Fusarium vasinfectum, Penicillium* species, *Cladosporium cladosporioides, Stemphylium languinosum, Rhizopus oryzae, Candida glabrata, Saccharomyces cerevisiae, Schizophyllum commune,* and *Trichosporon beigelii,*
- a bronchiectasis,
- an emphysema, or
- chronic obstructive airway disease or chronic obstructive pulmonary disease (COPD).

In alternative embodiments, the Amphotericin B is administered by inhalation wherein the administration by inhalation comprises administration of the therapeutic formulation as a spray, an aerosol or a powder.

In alternative embodiments of the therapeutic combination of drugs for use in the methods:
(a) the at least one antifungal agent or composition or equivalent antifungal agent or composition and the first, second, third and/or fourth or more antibiotic or antibacterial agents are administered or taken together;
(b) the at least one antifungal agent or composition or equivalent antifungal agent or composition is administered before the first, second, third and/or fourth or more antibiotic or antibacterial agents; or
(c) the at least one antifungal agent or composition or equivalent antifungal agent or composition is administered after the first, second, third and/or fourth or more antibiotic or antibacterial agents.

In alternative embodiments of the therapeutic combination of drugs for use in the methods: the at least one antifungal agent or composition or equivalent antifungal agent or composition and the first, second, third and/or fourth or more antibiotic or antibacterial agents, are administered in pulsed dosages, optionally, comprising starting at a high dose, then a low dose, then a high dose, then a low dose.

In alternative embodiments of the therapeutic combination of drugs for use in the methods: the methods further comprise use of or administration of one or more of a mucolytic agent, a steroid, a decongestant and/or a bronchodilator.

In alternative embodiments of the therapeutic combination of drugs for use in the methods: a therapeutically effective combination of Amphotericin B, rifabutin, and azithromycin is administered.

In alternative embodiments of the methods administration is oral.

In alternative embodiments of the methods administration is by inhalation.

The details of one or more embodiments of the invention are set forth in the accompanying description below. Other features, objects, and advantages of the invention will be apparent from the description and the claims.

### BRIEF DESCRIPTION OF THE DRAWINGS

**FIG. 1** is a schematic representation of a cochleate. The insert depicts the lipid strata of the cochleate, which contains a phospholipid bilayer (circles and tails), multivalent cation (unshaded circles) and an exemplified cargo moiety (hatched circles) protected within the cochleate.
**FIG. 2** depicts a schematic of a macrophage engulfing a cochleate and its cargo. The insert depicts the opening of the cochleate and release of the cargo inside the macrophage as described in the detailed description.
**FIG. 3** depicts an exemplary preparation of geode cochleates as described in the detailed description.

### DETAILED DESCRIPTION

In alternative embodiments, the therapeutic combinations may be for use in methods for treating, amelioriating, reversing and/or preventing (acting as a prophylaxis): a respiratory condition, or any lung condition, involving infection and/or inflammation of a respiratory mucosa, and/or infection and/or inflammation of an underlying muscle of the respiratory tract; an asthma; a bronchitis; a sinusitis or rhinosinusitis; an infection of a sinus; or, a bronchiectasis. In alternative embodiments, the therapeutic combination comprises: a combination of an Amphotericin B, and: two antibiotics; three antibiotics; or, four or more antibiotics; wherein the therapeutic combination comprises azithromycin, and rifabutin.

In alternative embodiments, the therapeutic compositions are formulated into delivery or storage vehicles, e.g., capsules, pills, tablets, geltabs, in e.g., a powder, e.g., a lyophilised, format; or alternatively, are formulated as liquids, e.g., as a liquid drink, for those who cannot swallow capsules.

In alternative embodiments, provided are effective treatments for respiratory mucosal inflammation to overcome the shortcomings of the previous art in the area. For example, in alternative embodiments not forming part of the invention, provided are therapeutic compositions and methods using oral Amphotericin B combined with antibiotic agents such as rifabutin, clarithromycin or doxycycline, or any other antibiotics, e.g., antibiotics or combinations thereof designed to also treat the *Chlamydia pneumoniae* infections, *Haemophilus influenzae* and *Mycoplasma pneumoniae,* which often co-exist in the lungs together with the fungal infection.

In alternative embodiments, the at least one antifungal agent or composition or equivalent antifungal agent or composition and the first, second, third and/or fourth or more antibiotic or antibacterial agents are administered in pulsed dosages, optionally, comprising starting at a high dose, then a low dose, then a high dose, then a low dose, etc., until a physician determines an appropriate endpoint for administration.

In alternative embodiments, the term "treatment" refers to any and all uses which remedy a disease or sate, relieve or abrogate a symptom, or otherwise prevent hinder, retard, or reverse the progression of a disease or a rather undesirable symptom in anyway whatsoever. In alternative embodiments, "treatment" means not only treatment designed to cure to remove symptoms in an individual, but also to ongoing therapy (so-called 'maintenance therapy') designed to control and suppress the recurrence of symptoms due to regrowth of a pathogen or infectious agent, e.g., a bacterium. In alternative embodiments, treatment is for a defined period of time, or provided there is ongoing basis of treatment depending on particular circumstances in any given individual, the treatment might be cyclic, sequential, combined or given in varying doses for particular times.

In alternative embodiments, the term "inflammation" refers parts of the complex biological response of body tissues to harmful stimuli, such as pathogens, damaged cells, or irritants, and is a protective response involving immune cells, blood vessels, and molecular mediators. The function of inflammation is to eliminate the initial cause of cell injury, clear out necrotic cells and tissues damaged from the original insult and the inflammatory process, and initiate tissue repair. Inflammation includes acute and chronic inflammation. Acute inflammation refers to the initial response of the body to harmful stimuli and is achieved by increased movement of harmful stimuli of plasma and leukocytes from blood into injured tissues. Chronic inflammation refers to prolonged inflammation leading to a progressive shift in the type of cells present at the site of inflammation and is characterized by simultaneous destruction and healing of the tissue from the inflammation process.

In alternative embodiments, the term "bronchitis" refers to inflammation of the bronchi in the lungs. Bronchitis includes both acute and chronic bronchitis. Chronic bronchitis is characterized by a productive cough that lasts for three months or more per year for at least two years.

In alternative embodiments, the term "bronchiectasis" refers to a disease in which there is permanent enlargement of one or more parts of the airways of the lung.

The term "sinusitis" or "rhinosinusitis" refers to inflammation of one or more sinuses resulting in infection. The one or more sinuses can include, but is not limited to, the paranasal sinuses, e.g., the frontal, ethmoidal (such as the anterior and posterior ethmoidal sinuses), maxillary, and sphenoidal sinuses.

In variants of the disclosure, Amphotericin B may be given orally (alone or in combination with another drug, e.g., an antibiotic) for one to ten days - then followed by an increase of the initial dose for an about a ten-day period - then followed by a further increase in dosage for another ten-day cycle, then a further increase in dosage for another ten-day cycle.

In variants of the disclosure, Amphotericin B may be given orally (alone or in combination with another drug, e.g., an antibiotic) for one to ten days - then followed by a decrease of the initial dose for an about a ten-day period - then followed by a further decrease in dosage for another ten-day cycle, then a further decrease in dosage for another ten-day cycle.

In alternative embodiments, the term "anti-bacterial agent" or "antibiotic" refers to any agent that is capable of killing a bacterium or an infectious agent, or is used in the treatment or eradication of infections caused by bacteria or infectious agents. This includes both antibiotics isolated from natural sources and synthetically generated anti-bacterials.

In alternative embodiments, the term "emollient" refers to any product applied to the skin which soothes irritation of the skin, including, for example, ointments, liniments, lotions, creams, moisturisers, oils, skin softeners, soaps, shampoo, sunscreens, cosmetics and the like.

In alternative embodiments, the term "simultaneously" means administration within a 0.1, 0.5, 1, 2, 5, 10 or 24-hour period; for example, to realise the benefits of exemplary embodiments as provided herein it is not necessary that administration of each of the active agents (e.g., antifungals, antibiotics) occur at precisely the same time, but rather that the individual receive these agents within a given 0.1, 0.5, 1, 2, 5, 10 or 24-hour period.

### Oral Formulations of Amphotericin B and equivalents - Not forming part of the invention

Provided are therapeutic compositions, and methods comprising use of, oral forms of Amphotericin B (AMB), including lipid based formulations such as mixtures of mono and diglycerides with phospholipids. These lipid-based formulations enhance Amphotericin B solubility, see, e.g., formulations of Amphotericin B by iCO Therapeutics Inc. and Matinas BioPharma.

In alternative variants, an encochleated formulation of Amphotericin B is used (Matinas BioPharma, Inc.). In alternative variants, the cochleates have a multilayer crystalline, spiral structure with no internal aqueous space; the structure is formed when a series of solid lipid sheets roll up and capture drug molecules in between the sheet, a process referred to as "encochleation."

In alternative variants, encochleation involves combining calcium and soy-derived phospholipids (PS), two naturally occurring materials classified as GRAS (generally recognized as safe) by the FDA, through a tightly controlled crystallization process to envelop the Active Pharmacological Ingredient (API). The result is a lipid-crystal encochleated drug formulation, such as described in International Patent Application Publication Nos. WO 2017/205550 and WO 2018/013711.

Cochleates are anhydrous, stable, multi-layered lipid crystals which spontaneously form upon the interaction of negatively charged lipids, such as phosphatidylserine, and divalent cations, such as, calcium (see, for example, U.S. Pat. Nos. 4,078,052; 5,643,574; 5,840,707; 5,994,318; 6,153,217; 6,592,894, as well as PCT Publ. Nos. WO 2004/091572; WO 2004/091578; WO 2005/110361, WO 2012/151517, and WO2014/022414, and U.S. Pat. Publ. 2010/0178325. Typically, these are referred to as "crystal cochleates."

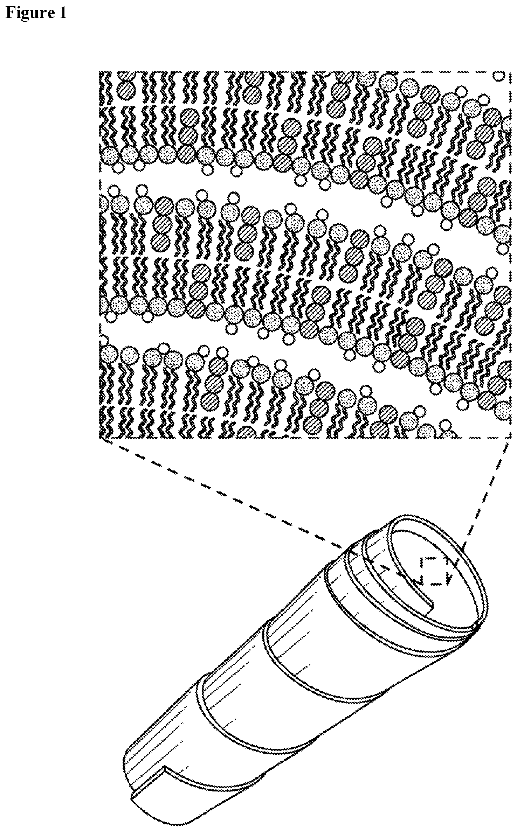

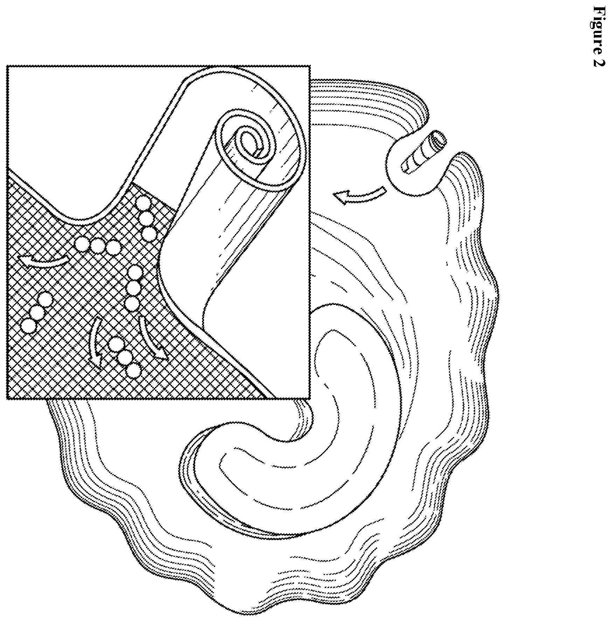

Crystal cochleates have a unique multilayered structure consisting of a large, continuous, solid, phospholipid bilayer sheet or strata rolled up in a spiral or as stacked sheets, with no internal aqueous space (FIG. 1). This unique structure provides protection from degradation for associated "encochleated" molecules. Since the entire cochleate structure is a series of solid layers, components within the interior of the cochleate structure remain intact, even though the outer layers of the cochleate may be exposed to harsh environmental conditions or enzymes. Divalent cation concentrations in vivo in serum and mucosal secretions are such that the cochleate structure is maintained. Hence, the majority of cochleate-associated molecules are present in the inner layers of a solid, stable, impermeable structure. Once within the interior of a cell, however, the low calcium concentration results in the opening of the cochleate crystal and release of the molecule that had been formulated into cochleates (FIG. 2). Accordingly, cochleate formulations remain intact in physiological fluids, including mucosal secretions, plasma and gastrointestinal fluid, thereby mediating the delivery of biologically active compounds by many routes of administration, including intramuscular and mucosal, e.g., intranasal and oral.

Typical cochleate structures include a lipid strata comprising alternating divalent cations and phospholipid bilayers that include at least one negatively charged phospholipid. Typically, a cargo moiety, such as a bioactive agent as described herein, is sequestered within the lipid strata of the cochleate.

In another variants, the cochleates of the present disclosure are formed using the DC (Direct Calcium) Dialysis method. In this method, detergent is removed from a solution of lipid and material to be encochleated by dialysis against a buffer containing multivalent cation. The removal of detergent and addition of multivalent cation therefore take place simultaneously, rather than sequentially as in the LC method. In some implementations, this method is used in the formulation of immunogenic compositions of the disclosure, such as vaccines, containing nucleic acids, such as DNA plasmids. The DC method is also described in U.S. Patent No. 5,994,318.

As recognized by an ordinary artisan, many parameters, including pH, salt concentration, agitation method and rate, cation type, concentration, and rate of addition, lipid composition, concentration, and ratio of lipid to other material, etc., affect the formulation, and can be varied in order to optimize the encochleation of a particular material.

In a typical implementation, the multivalent cation is a divalent metal cation, such as calcium, zinc, magnesium, and barium. In a more typical implementation, the divalent metal cation is calcium.

The liposome used during the formation of the cochleates may be multilamellar (MLV) or unilamellar (ULV), including small unilamellar vesicles (SUV). The concentration of lipid in these liposomal solutions can be from about 0.1 mg/mL to 500 mg/mL. Typically, the concentration of lipid is from about 0.5 mg/mL to about 50 mg/mL, more typically from about 1 mg/mL to about 25 mg/mL.

A size-regulating agent may be introduced during the method of making the cochleate. A size-regulating agent, as used herein, refers to an agent that reduces the particle size of a cochleate. As used herein, the term "particle size" refers to the particle diameter, or in case the particles are not spherical, to the largest extension in one direction of the particle.

The particle size of cochleates can be measured using conventional methods, such as a submicron particle size analyzer. In certain variants, the size regulating agent is a lipid-anchored polynucleotide, a lipid-anchored sugar (glycolipid), or a lipid-anchored polypeptide. In other variants, the size regulating agent is a bile salt, such as oxycholate, cholate, chenodeoxycholate, taurocholate, glycocholate, taurochenodeoxycholate, glycochenodeoxy cholate, deoxycholate, or lithocholate. Bile salts are bile acids compounded with a cation, usually sodium. Bile acids are steroid acids found predominantly in the bile of mammals and are commercially available.

In certain variants, the size-regulating agent is added to the lipid or liposomes before formation of the precipitated cochleate. For example, in one embodiment, the size-regulating agent is introduced into a liposomal suspension from which cochleates will subsequently be formed (e.g., by addition of cation or dialysis). Alternatively, the size-regulating agent may be introduced to a lipid solution, before or after addition of a pharmacologically active agent.

Any suitable lipid can be used to make the cochleate. In one variant, the lipid includes one or more negatively charged lipids. As used herein, the term "negatively charged lipid" includes lipids having a head group bearing a formal negative charge in aqueous solution at an acidic, basic or physiological pH, and also includes lipids having a zwitterionic head group. In one variant, the negatively charged lipid is a phospholipid.

The cochleates can also include non-negatively charged lipids (e.g., positive and/or neutral lipids). Typically, the cochleates include a significant amount of negatively charged lipids. In certain variants, a majority of the lipid is negatively charged. In one embodiment, the lipid is a mixture of lipids, comprising at least 50% negatively charged lipid, such as a phospholipid. In another variant, the lipid includes at least 75% negatively charged lipid, such as a phospholipid. In other variants, the lipid includes at least 85%, 90%, 95% or 98% negatively charged lipid, such as a phospholipid. In yet other variants, the negatively charged lipid (e.g., phospholipid) comprises between 30%-70%, 35%-70%, 40%-70%, 45%-65%, 45%-70%, 40%-60%, 50%-60%, 45%-55%, 45%-65%, or 45%-50% of the total lipid in the cochleate. In certain variants, the negatively charged lipid (e.g., phospholipid) comprises between 40%-60% or 45%-55% of the total lipid in the cochleate. In some variants, the negatively charged lipid (e.g., phospholipid) comprises between 30%-70%, 35%-70%, 40%-70%, 45%-65%, 45%-70%, 40%-60%, 50%-60%, 45%-55%, 45%-65%, or 45%-50% of the total lipid in the non-hydrophobic domain component of the cochleate. In certain variants, the negatively charged lipid (e.g., phospholipid) comprises between 40%-60% or 45%-55% of the total lipid in the non-hydrophobic domain component of the cochleate. In some variants, the negatively charged lipid is a phospholipid and comprises between 30%-70%, 35%-70%, 40%-70%, 45%-65%, 45%-70%, 40%-60%, 50%-60%, 45%-55%, 45%-65%, or 45%-50% of the total phospholipid in the cochleate or in the non-hydrophobic domain component of the cochleate. In some variants, the negatively charged lipid is a phospholipid and comprises about 40%-60% or 45%-55% of the total phospholipid in the cochleate or in the non-hydrophobic domain component of the cochleate.

The negatively charged lipid can include egg-based lipids, bovine-based lipids, porcine-based lipids, plant-based lipids such as soy-based lipids, or similar lipids derived from other sources, including synthetically produced lipids. The negatively charged lipid can include phosphatidylserine (PS), dioleoylphosphatidylserine (DOPS), phosphatidic acid (PA), phosphatidylinositol (PI), and/or phosphatidyl glycerol (PG) and or a mixture of one or more of these lipids with other lipids. Additionally or alternatively, the lipid can include phosphatidylcholine (PC), phosphatidylethanolamine (PE), diphosphotidylglycerol (DPG), dioleoyl phosphatidic acid (DOPA), distearoyl phosphatidylserine (DSPS), dimyristoyl phosphatidylserine (DMPS), dipalmitoyl phosphatidylglycerol (DPPG) and the like. In another embodiment, the phosphatidylserine is egg or bovine derived phosphatidylserine.

In some variants, the cochleates, including the geode cochleates, described herein below, are prepared using legume-based phospholipids, more typically soy-based lipids. Such soy-based lipids can be natural or synthetic. Even more typically, the soy-based lipids are soy phospholipids, such as soy phosphatidylserine is in an amount of about 40%-74% by weight of the lipid component of the cochleates. Alternatively, the soy phosphatidylserine can be about 40%, 45%, 50%, 55%, 60%, 65% or 70% or any incremental value thereof, by weight of the lipid component of the cochleates. It is to be understood that all values, and ranges between these values and ranges are meant to be encompassed by the present disclosure. In a typical variant, the phospholipid comprises about 45-70% soy phosphatidylserine. In a more typical variant, the phospholipid comprises about 45-55% soy phosphatidylserine.

Soy phosphatidylserine is commercially available, e.g., from Avanti Polar Lipids, Inc. Alabaster, AL. Alternatively, soy phosphtidylserine can be purified from soy phospholipid compositions, which are mixtures of several soy phospholipids, according to well-known and standard purification techniques.

In some variants, neutral lipids are used in combination with the soy phosphatidylserine to make the instant cochleates. As used herein, the term "neutral lipids" include any of a number of lipid species, which exist either in an uncharged or neutral zwitterionic form at physiological pH and, thus, are included within the group of lipids lacking an anionic function. Such lipids include, for example diacylphosphatidylcholine, diacylphosphatidylethanolamine, ceramide, sphingomyelin, dihydrosphingomyelin, cephalin, and cerebrosides. The selection of neutral lipids for use in the cochleate compositions described herein is generally guided by consideration of, e.g., cochleate size and stability. Lipids having a variety of acyl chain groups of varying chain length and degree of saturation are available or may be isolated or synthesized by well-known techniques. In one group of variants, lipids containing saturated fatty acids with carbon chain lengths in the range of C14 to C22 can be used. In another group of variants, lipids with mono- or diunsaturated fatty acids with carbon chain lengths in the range of C14 to C22 can be used. In yet another group of embodiments, lipids with mono- or diunsaturated fatty acids with carbon chain lengths in the range of C8 to C12 can be used. Additionally, lipids having mixtures of saturated and unsaturated fatty acid chains can be used.

In some variants, the neutral lipids used in the present disclosure are DOPE, DSPC, DPPC, POPC, or any related phosphatidylcholine. The neutral lipids useful in the present disclosure may also be composed of sphingomyelin, dihydrosphingomyeline, or phospholipids with other head groups, such as serine and inositol.

In a typical implementation, 99.9% pure dioleoyl phosphatidylserine, 99.9% pure soy phosphatidylserine, 75% soy phosphatidylserine and 50% soy phosphatidylserine, are used to manufacture cochleates. The lipid composition of 99.9% pure phosphatidylserine is typically modified by the addition of neutral lipids, including, but not limited to sphingomyelin and/or phosphatidylcholine. When lower purity phosphatidylserine (e.g., 50% soy phosphatidylserine) is used as a starting material, the lower purity phosphatidylserine can be subjected to extraction steps to remove unwanted impurities, such as, nucleases.

In some implementations, the cochleate of the present disclosure is a geode cochleate, or a geodate, as described, for example, in U.S. Patent Publication 2013/0224284. Geode cochleates further comprise a lipid monolayer comprising a negatively charged phospholipid, where the lipid monolayer surrounds a hydrophobic domain, such as an oil, and a cargo moiety, such as a bioactive agent as described herein, which is dispersed within the hydrophobic domain. The lipid monolayer is sequestered within the lipid strata of the geode cochleate.

As used herein, a "hydrophobic domain" is a composition that is sufficiently hydrophobic in nature to allow formation of a lipid monolayer about its periphery. A hydrophobic domain typically includes a hydrophobic composition, such as oil or fat, associated with a cargo moiety. In certain variants, the ratio between the hydrophobic domain (HD) and the phospholipid component of the geode cochleate (PPLGD) HD:PPLGD or the castor oil domain (COD) and phospholipid component of the geode cochleate (PPLGD) COD:PPLGD is about 1 :20 or less, 1 : 15 or less, 1 : 10 or less, 1 :8 or less, 1 :6 or less, 1 :5 or less, 1 :4 or less, 1 :3.5 or less, 1 :3 or less, 1 :2.75 or less, 1 :2.5 or less, 1 :2.25 or less, 1 :2 or less, 1 : 1.75 or less, 1 : 1.5 or less, 1 : 1.25 or less 1 : 1 or less.

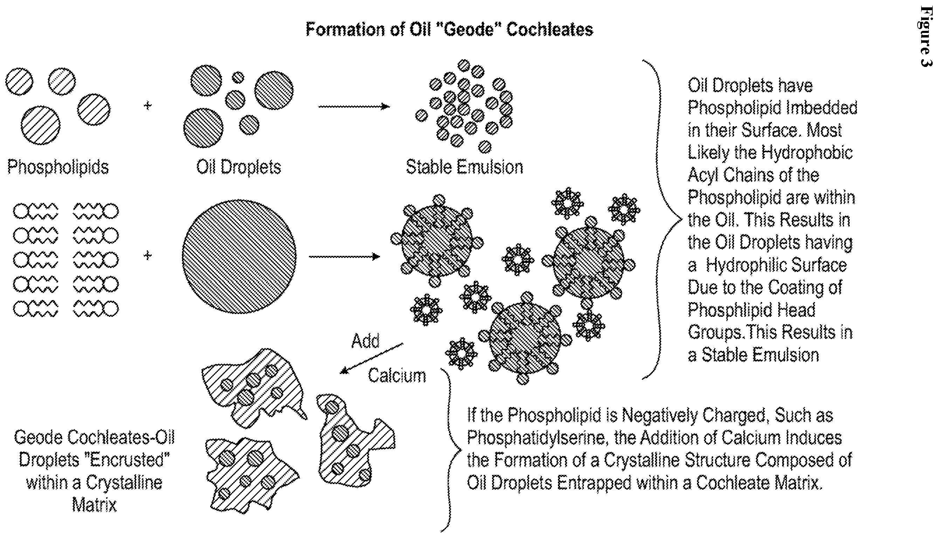

FIG. 3 shows an exemplary schematic of how geode cochleates can be made. In this exemplary method, a phospholipid (represented as an open ring) is combined with a hydrophobic domain (shaded circles), such as an oil, and mixed to form a stable emulsion comprising liposomes and lipid monolayers surrounding the hydrophobic domain. A cargo moiety may be dispersed within the hydrophobic domain. The hydrophobic domains have phospholipids imbedded in their surface. Without intending to be bound by any theory, it is believed that the hydrophobic acyl chains of the phospholipid are within the hydrophobic domains, resulting in the hydrophobic domains having a hydrophilic surface due to the coating of the phospholipid head groups and forming a stable emulsion. If the phospholipid is negatively charged, such as with phosphatidylserine, the addition of a divalent cation, such as calcium, induces the formation of a crystalline structure (or lipid strata) comprising alternating divalent cations and phospholipid bilayers. The lipid strata are represented with hatching. In a geode cochleate, the lipid monolayers surrounding the hydrophobic domain are "encrusted" or "entrapped" within the crystalline matrix, akin to a "geode."

Encochleated formulations according to the present invention include lipid crystals made up of nano-sized particles of about 10 to 1000 nanometers in diameter, or about 20 to 500 nanometers in diameter, or about 50 to 100 nanometers in diameter. In alternative embodiments, encochleated drug formulations of the present invention are formulated for mucosal (e.g., oral or intranasal) administration or may be administered mucosally.

In alternative embodiments, any nano-suspension delivery system can be used, e.g., to result in significantly increased Amphotericin B (AMB) solubility.

In alternative embodiments, oral forms of Amphotericin B are used. It is believed that oral forms of Amphotericin B as described herein may avoid the renal toxicity of other parenteral Amphotericin B.

In alternative embodiments, orally formulated Amphotericin B used alone or in the therapeutic combinations as provided herein comprises one or more of:
- a FUNGILIN^{™} (Aspen Pharma Pty Ltd, New Zealand; iCo Therapeuics Inc., Vancouver, BC, Canada), optionally as tablets, suspensions or lozenges;
- a micronized formulation of Amphotericin B;
- a nanosuspension of Amphotericin B, optionally made by high-pressure homogenization, e.g., as described by Wasan et al, J. of Infectious Disease (2009) vol 200(3):357-360), or as described by Torrado et al Therapeutic Delivery (2012) vol 4(1):9-12, optionally the nanosuspension comprising Tween 80 (optionally at 0.5% w/w), Pluronic F68 (optionally at 0.25% w/w), and sodium cholate (optionally 0.05% w/w);
- a solubilized formulation of Amphotericin B, e.g., as described by Kravetz et al N. Engl. J. Med (1961) 265:183-184;
- Amphotericin B nanoparticles, optionally comprising PEGylated polylactic-polyglycolic acid copolymer (PLGA-PEG) nanoparticles (NPs) or equivalents, e.g., as described by Radwan et al, J. Drug Delivery (2017) vol 24(1):40-50;
- Amphotericin B attached to functionalized carbon nanotubes, e.g., as described in Torrado et al Therapeutic Delivery (2012) vol 4(1):9-12
- a lipid-based formulation of Amphotericin B comprising mono- and diglycerides with phospholipids; and/or
- an encochleated (a lipid-crystal nano-particle formulation) formulation of Amphotericin B, optionally MAT2203 (Matinas Biopharma Laboratories/ Matinas BioPharma Nanotechnologies, Inc., Bridgewater, NJ).

### Administration and formulations

According to the invention, Amphotericin B is formulated for administration by inhalation. In alternative embodiments, administration of therapeutic combinations as provided herein, including anti-fungal and anti-bacterial agents, may be by oral, intravenous, intra-arterial, intramuscular, or subcutaneous routes.

In alternative embodiments, the at least one anti-fungal agent and at least one anti-bacterial agent may be administered in single daily doses, or in two, three, four or more identical or different divided doses per day, and they may be administered simultaneously or at different times during the day. In alternative embodiments, the active substances (e.g., anti-fungal and anti-bacterial agents) are administered simultaneously. In alternative embodiments, active substances (e.g., anti-fungal and anti-bacterial agents) are contained in separate medications or formulations, e.g., separate capsules or tablets, or alternatively, in a single combined dosage form, e.g., a single capsule or tablet.

In alternative embodiments, doses of exemplary anti-fungal agents and anti-bacterial agents as used in the pharmaceutical compositions and therapeutic combinations as provided herein are accordance with their generally known and established safe dosage ranges when they are used in monotherapy for the treatment of other conditions. Such dosages for anti-bacterial agents are understood by those skilled in the art and generally range from 0.0005 to 50 grams per day, depending on the agent used, as described for example in Martindale, The Extra Pharmacopoeia, 31st Edition (The Royal Pharmaceutical Society, London, 1996).

In alternative embodiments, the therapeutically effective amount of anti-fungal and anti-bacterial agents for any particular patient will depend upon a variety of factors including: the disorder being treated and the severity of the disorder; the composition employed; the age, body weight, general health, sex and diet of the patient; the time of administration; the route of administration; the duration of the treatment; drugs used in combination or coincidental with the treatment, together with other related factors well known in medicine.

In alternative embodiments, one skilled in the art would be able, by routine experimentation, to determine an effective, non-toxic amount of the anti-fungal and anti-bacterial agents which would be required to treat the disorders and diseases for which the therapeutic combinations as provided herein are administered.

In alternative embodiments, an effective dose of an exemplary anti-fungal agent(s) and anti-bacterial agent(s), or Amphotericin B or equivalent alone, are in the range of about 1 milligram (mg) per day to about 4 grams (g) per day, alternatively about 10 mg per day to about 2 g per day, alternatively about 100 mg per day to about 1000 mg per day. In alternative embodiments of therapeutic combinations and methods as provided herein, a patient is administered Amphotericin B or equivalent alone (e.g., as the only active antimicrobial agent, while other active drugs, emollients, buffers, carriers, excipients, and the like may also be present) in an amount of about 250 mg per day.

Exemplary anti-fungal agents and anti-bacterial agents include, but are not limited to, flucytosine, ketoconazole, miconazole, itraconazole, fluconazole, griseofulvin, clotrimazole, econazole, terconazole, butoconazole, oxiconazole, sulconazole, supraconazole, voriconazole, ciclopirox olamine, haloprogin, tolnaftate, naftifine, terbinafine hydrochloride, a morpholine, nystatin, natamycin, butenafine, undecylenic acid, proprionic acid, caprylic acid, tetracyclines, penicillins, macrolides, quinolones, chloramphenicol, rifamycins, sulphonamides, co-trimoxazole, oxazolidinones, a doxycycline, chlortetracycline, tetracycline hydrochloride, oxytetracycline, demeclocycline, methacycline, minocycline, penicillin, amoxycillin, erythromycin, clarithromycin, roxithromycin, azithromycin, spiramycin, oleandomycin, josamycin, kitsamysin, flurithromycin, nalidixic acid, oxolinic acid, norfloxacin, perfloxacin, amifloxacin, ofloxacin, ciprofloxacin, sparfloxacin, levofloxacin, rifabutin, rifampicin, rifapentin, sulfisoxazole, sulfamethoxazole, sulfadiazine, sulfadoxine, sulfasalazine, sulfaphenazole, dapsone, sulfacytidine, linezolid, acetylsalicylic acid, fenticonazole, isoconazole, luliconazole, omoconazole, sertaconazole, tioconazole, albaconazole, efinaconazole, epoxiconazole, isavuconazole, posaconazole, propiconazole, ravuconazole, and terconazole.

In variants of therapeutic combinations and methods as provided herein, not according to the invention, a patient is administered Amphotericin B or equivalent in an amount of about 250 mg per day, doxycycline in an amount of about 100 mg per day and rifabutin in an amount of about 150 mg per day. In alternative embodiments, administration is oral. In alternative embodiments, administration is about twice (bid), three times (tid) or more daily, alternatively for a period of between one week and six months.

In variants of therapeutic combinations and methods as provided herein, not according to the invention, a patient is administered Amphotericin B or equivalent (about 250 mg per day), clarithromycin (about 500 mg per day) and rifampicin (about 300 mg per day). Alternatively, administration is oral, and/or twice daily; such a method of treatment, adjusted to reflect the child's weight, is particularly suitable for administration to children. An alternative effective treatment for children involves the administration of a syrup containing one anti-fungal agent in the form of terbinafine hydrochloride and one anti-bacterial agent in the form of clarithromycin.

In variants of therapeutic combinations and methods as provided herein, not according to the invention, a patient is administered Amphotericin B or equivalent (about 250 mg per day), doxycycline (about 100 mg per day) and rifampicin (about 300 mg per day). Alternatively, administration is oral, twice daily for a period of two months.

In variants of therapeutic combinations and methods as provided herein, not according to the invention, a patient is administered Amphotericin B or equivalent (about 250 mg per day), clarithromycin (about 250 mg per day) and rifabutin (about 150 mg per day). In alternative embodiments, the administration is oral, twice daily for a period of 1 week to 1 year.

In variants of therapeutic combinations and methods as provided herein, not according to the invention , a patient is administered Amphotericin B or equivalent (about 250 mg per day), tetracycline hydrochloride (about 500 mg per day) and rifampicin (about 300 mg per day). Preferably administration is oral, twice daily for a period of 1 to 3 months.

In variants of therapeutic combinations and methods as provided herein, not according to the invention, a patient is administered Amphotericin B or equivalent (about 200 mg per day), clarithromycin (about 500 mg per day) and rifabutin (about 150 mg per day), alternatively administration is oral, twice daily for a period of 1 to 3 months.

In variants of therapeutic combinations and methods as provided herein, not according to the invention, a patient is administered Amphotericin B or equivalent (about 250 mg per day), erythromycin (about 1000 mg per day) and amoxicillin (about 1000 mg per day). Alternatively, administration is oral, twice daily.

In variants of therapeutic combinations and methods as provided herein, not according to the invention, a patient is administered Amphotericin B or equivalent (about 500 mg per day), clarithromycin (about 500 mg per day) and doxycycline (about 100 mg per day). Alternatively, administration is oral, twice daily.

The Amphotericin B or equivalent antifungal agent may be formulated in any suitable amount of Amphotericin B or equivalent antifungal agent, such as from about 1 mg to about 4 g, or about 10 mg to about 2 g, or about 100 mg to about 1000 mg, or between about 200 mg to about 300 mg, or about 300 mg to about 500 mg, or about 100 mg, about 200 mg, about 250 mg, about 300 mg, about 350 mg, about 400 mg, or about 500 mg of Amphotericin B or equivalent antifungal agent.

It will be appreciated by those skilled in the art that the dosages provided in the above exemplary embodiments are merely indicative of typical dosages of the specific anti-fungal agents and anti-bacterial agents listed. Actual doses of each active agent administered to any given individual may vary and will depend on a variety of factors including: the disorder being treated and the severity of the disorder; the composition employed; the age, body weight, general health, sex and diet of the individual; the time of administration; the route of administration; the duration of the treatment; drugs used coincidental with the treatment, together with other related factors well known in medicine. For example, dosage amounts higher than those provided herein may be employed in the case of resistant fungal and bacterial infections.

In alternative embodiments, administration of the relevant composition will be determined on a case by case basis, and may for example be once, twice, three or more times daily. In alternative embodiments, component medications are taken simultaneously or separately. In alternative embodiments, duration of the treatment will depend on the severity and resistance of the underlying condition. In alternative embodiments, treatment is prescribed for a duration of one week to one year or more, optionally for about one to four, or for about two to three months.

In alternative embodiments, patient response to the treatment is measured by noting e.g., clinical improvement, progressive reduction of reliance upon typical asthma drugs such as steroids and bronchodilators, the improvement in peak flow, and patient well-being and performance.

In alternative embodiments, individual ingredients of or the therapeutic combinations as provided herein, are formulated as pharmaceutical compositions, and may include one or more pharmaceutically acceptable excipients, adjuvants, diluents or carriers which are generally known in the art.

In alternative embodiments, for oral administration, the pharmaceutical compositions are in the form of tablets, lozenges, pills, troches, capsules, elixirs, powders, including lyophilised powders, solutions, granules, suspensions, emulsions, syrups and tinctures. In alternative embodiments, slow release, or sustained release forms can be prepared, for example in the form of coated particles, multi-layer tablets or microgranules. In alternative embodiments, for prolonged action the composition can be in a slow or sustained release form.

In alternative embodiments, solid forms for oral administration may contain pharmaceutically acceptable binders, sweeteners, disintegrating agents, diluents, flavourings, coating agents, preservatives, lubricants and/or time delay agents. Suitable binders include gum acacia, gelatin, corn starch, gum tragacanth, sodium alginate, carboxymethylcellulose or polyethylene glycol. Suitable sweeteners include sucrose, lactose, glucose, aspartame or saccharine. Suitable disintegrating agents include corn starch, methylcellulose, polyvinylpyrrolidone, xanthan gum, bentonite, alginic acid or agar. Suitable diluents include lactose, sorbitol, mannitol, dextrose, kaolin, cellulose, calcium carbonate, calcium silicate or dicalcium phosphate. Suitable flavouring agents include peppermint oil, oil of wintergreen, cherry, orange or raspberry flavouring. Suitable coating agents include polymers or copolymers of acrylic acid and/or methacrylic acid and/or their esters, waxes, fatty alcohols, zein, shellac or gluten. Suitable preservatives include sodium benzoate, vitamin E, alpha-tocopherol, ascorbic acid, methyl paraben, propyl paraben or sodium bisulphite. Suitable lubricants include magnesium stearate, stearic acid, sodium oleate, sodium chloride or talc. Suitable time delay agents include glyceryl monosterate or glyceryl disterate.

In alternative embodiments, liquid forms for oral administration may contain in addition to the active agents, a liquid carrier. Suitable liquid carriers include water, oils such as olive oil, peanut oil, sesame oil, sunflower oil, safflower oil, arachis oil, coconut oil, liquid paraffin, ethylene glycol, propylene glycol, polyethylene glycol, ethanol, propanol, isopropanol, glycerol, fatty alcohols, triglycerides or mixtures thereof.

In alternative embodiments, suspensions for oral administration may further include dispersing agents and/or suspending agents. Suitable suspending agents include sodium carboxymethylcellulose, methylcellulose, hydroxypropylmethyl-cellulose, poly-vinyl-pyrrolidine, sodium alginate or cetyl alcohol. Suitable dispersing agents include lecithin, polyoxyethylene esters of fatty acids such as stearic acid, polyoxyethylene sorbitol mono- or di-oleate, -stearate or laurate, polyoxyethylene sorbitan mono- or di-oleate, -stearate or - laurate and the like.

In alternative embodiments, therapeutic combinations or pharmaceutical compositions as provided herein are prepared by blending, grinding, homogenising, suspending, dissolving, emulsifying, dispersing and/or mixing the anti-fungal agent(s) and the anti-bacterial agent(s) together with the selected excipient(s), carrier(s), adjuvant(s) and/or diluent(s). In alternative embodiments, a pharmaceutical composition is in the form of a tablet or capsule and can be prepared by, e.g., (a) preparing a first tablet comprising at least one of the active substances together with any desired excipient(s), carrier(s), adjuvant(s) and/or diluent(s), and (b) preparing a second tablet or a capsule, wherein the second tablet or the capsule includes the remaining active substance(s) and the first tablet.

In alternative embodiments, a therapeutic combination or pharmaceutical composition as provided herein is in the form of a capsule and can be prepared by (a) preparing a first capsule comprising at least one of the active substances together with any desired excipient(s), carrier(s), adjuvant(s) and/or diluent(s), and (b) preparing a second capsule, wherein the second capsule includes the remaining active substance(s) and the first tablet.

In alternative embodiments, a therapeutic combination or pharmaceutical composition as provided herein is in the form of a tablet and can be prepared by (a) preparing a capsule comprising at least one of the active substances together with any desired excipient(s), carrier(s), adjuvant(s) and/or diluent(s), and (b) preparing a tablet, wherein the tablet includes the remaining active substance(s) and the capsule.

In variants of the disclosure, a therapeutic combination or a pharmaceutical composition as provided herein comprises at least one anti-fungal agent and at least one anti-bacterial agent may be provided with the active substances contained within a single capsule for monotherapeutic administration. In alternative embodiments, in one form of such a composition, the at least one anti-fungal agent may be contained within an inner capsule or tablet, the inner capsule or tablet being surrounded by the at least one anti-bacterial agent contained within an outer capsule. In alternative embodiments, the locations of the components may be reversed such that the at least one anti-bacterial agent may be contained within the inner capsule or tablet and the at least one anti-fungal agent may be contained within the outer capsule. This arrangement will be especially desirable if the active substances are likely to cross-react if contained within the same capsule.

In alternative embodiments, in compositions that comprise three active substances in the form of one anti-fungal agent and two anti-bacterial agents, one active substance may be contained within a central coated tablet or capsule and the remaining two active substances may be contained in an outer capsule in the form of coated microspheres.

Other combinations for presentation of the combination of three active substances are also provided.

### Multicomponent Packaging

Provided are multi-component delivery systems, e.g., products of manufacture, comprising e.g., the therapeutic combinations and formulations as provided herein, e.g., antifungals and anti-bacterials, e.g., as formulated and dosaged for oral administration, e.g., as capsules, tablets, geltabs, as a powder, e.g., a lyophilised powder, and another component, e.g., a liquid; these multi-component delivery systems, e.g., products of manufacture, can be designed or manufactured as described e.g., in USPNs 8,968,717; 8,931,665; 7,861,854; 7,018,089; and 6,626,912; and, U.S. Pat. App. Pub. nos. 2010/0034574; 2009/0180923; 20090232886; 2008/0160076; 2007/0087048; 2007/0036830; 2007/0074979; 2005/0205438; 2004/0089563.

### Packaging

In alternative embodiments, combinations of ingredients of therapeutic combinations as provided herein are mixed and administered together, or alternatively, they can be an individual member of a packaged combination of ingredients, e.g., a liquid component and a solid or powder component manufactured in separate compartments, packages, kits or containers; e.g., where all or a subset of the combinations of ingredients are manufactured in a separate compartment, package or container. In alternative aspects, the package, kit or container comprises a blister package, a clamshell, a tray, a shrink wrap and the like.

Multiple dosages of orally administered Amphotericin B or equivalent may be provided in one package, kit or container (e.g., a "blister package"), where the same or different dosages are included for once day, bid, tid or more times a day administration regimen to the patient, where the package, kit or container (e.g., a "blister package") clearly indicated when each day the dose is to be taken.

In one embodiment, the package, kit or container comprises a "blister package" (also called a blister pack, or bubble pack). In one embodiment, the blister package is made up of two separate elements: a transparent plastic cavity shaped to the product and its blister board backing. These two elements are then joined together with a heat sealing process which allows the product to be hung or displayed. Exemplary types of "blister packages" include: Face seal blister packages, gang run blister packages, mock blister packages, interactive blister packages, slide blister packages.

Blister packs, clamshells or trays are forms of packaging used for goods; thus, the invention provides for blister packs, clamshells or trays comprising a microbiota product used to practice the invention. Blister packs, clamshells or trays can be designed to be non-reclosable, so consumers can tell if a package has already been opened. They are used to package for sale goods where product tampering is a consideration, such as the pharmaceuticals of the invention. In one embodiment, a blister pack comprises a moulded PVC base, with raised areas (the "blisters") to contain the tablets, pills, etc. comprising the combinations of the invention, covered by a foil laminate. Tablets, pills, etc. are removed from the pack either by peeling the foil back or by pushing the blister to force the tablet to break the foil. In one embodiment, a specialized form of a blister pack is a strip pack. In one embodiment, in the United Kingdom, blister packs adhere to British Standard 8404.

Provided herein is a method of packaging where the compositions comprising combinations of ingredients, e.g., a therapeutic combination comprising a combination of Amphotericin B and two antibiotics or antibacterial agents, are contained in-between a card and a clear PVC. The PVC can be transparent so the item (pill, tablet, geltab, etc.) can be seen and examined easily; and in one embodiment, can be vacuum-formed around a mould so it can contain the item snugly and have room to be opened upon purchase. In one embodiment, the card is brightly colored and designed depending on the item (pill, tablet, geltab, etc.) inside, and the PVC is affixed to the card using pre-formed tabs where the adhesive is placed. The adhesive can be strong enough so that the pack may hang on a peg, but weak enough so that this way one can tear open the join and access the item. Sometimes with large items or multiple enclosed pills, tablets, geltabs, etc., the card has a perforated window for access. In one embodiment, more secure blister packs, e.g., for items such as pills, tablets, geltabs, etc. of the invention are used, and they can comprise of two vacuum-formed PVC sheets meshed together at the edges, with the informative card inside. These can be hard to open by hand, so a pair of scissors or a sharp knife may be required to open.

In one embodiment, blister packaging comprises at least two or three or more components (e.g., an antifungal and an antibacterial): a thermoformed "blister" which houses multi-ingredient combination of the invention, and then a "blister card" that is a printed card with an adhesive coating on the front surface. During the assembly process, the blister component, which is most commonly made out of PVC, is attached to the blister card using a blister machine. This machine introduces heat to the flange area of the blister which activates the glue on the card in that specific area and ultimately secures the PVC blister to the printed blister card. The thermoformed PVC blister and the printed blister card can be as small or as large as you would like, but there are limitations and cost considerations in going to an oversized blister card. Conventional blister packs can also be sealed (e.g., using an AERGO 8 DUO^{™}, SCA Consumer Packaging, Inc., DeKalb IL) using regular heat seal tooling. This alternative embodiment, using heat seal tooling, can seal common types of thermoformed packaging.

In alternative embodiments, an antifungal and/or an antibacterial can be formulated as, e.g., a powder, e.g., as lyophilised material, e.g., a lyophilized encapsulated product of the invention, e.g., for practicing methods of the invention, can be packaged alone or in combinations, e.g., as "blister packages" or as a plurality of packettes, including as lidded blister packages, lidded blister or blister card or packets or packettes, or a shrink wrap.

In alternative embodiments, laminated aluminum foil blister packs are used, e.g., for the preparation of orally administered therapeutic combinations as provided herein. Products or kits comprise an aqueous solution(s) that are dispensed (e.g., by measured dose) into containers. Trays can be freeze-dried to form tablets that take the shape of the blister pockets. The alufoil laminate of both the tray and lid fully protects any highly hygroscopic and/or sensitive individual doses. In one embodiment, the pack incorporates a child-proof peel open security laminate. In one embodiment, the system gives tablets an identification mark by embossing a design into the alufoil pocket that is taken up by the tablets when they change from aqueous to solid state. In one embodiment, individual 'push-through' blister packs/ packettes are used, e.g., using hard temper aluminum (e.g., alufoil) lidding material. In one embodiment, hermetically-sealed high barrier aluminum (e.g., alufoil) laminates are used. In one embodiment, any of the invention's products of manufacture, including kits or blister packs, use foil laminations and strip packs, stick packs, sachets and pouches, peelable and non-peelable laminations combining foil, paper, and film for high barrier packaging.

In alternative embodiments, provided herein are multi-component products of manufacture, including kits or blister packs that include memory aids to help remind patients when and how to take the therapeutic combination as provided herein. This safeguards the therapeutic agent's efficacy by protecting each tablet, geltab or pill until it's taken; gives the product or kit portability, makes it easy to take a dose anytime or anywhere.

The invention will be further described with reference to the examples described herein; however, it is to be understood that the invention is not limited to such examples.

### EXAMPLES

### Reference Example 1: Exemplary treatment of asthma using oral Amphotericin B

Adults having long-standing asthma, or symptomatic refractory asthma with demonstrated airway hyper-responsiveness to hypertonic saline, and/or a poor response to current steroid inhalants, are given an exemplary oral Amphotericin B formulation or formulations as a therapy, initially once a day, later twice daily, and then three times daily. In alternative embodiments, oral Amphotericin B is initially given bid or tid. Dosage is maintained until patient is able to cease using oral steroid inhalants, or at least is able to reduce steroid dosage by about 70%, or 80%, or 90%. Patients are continuously monitored for detectable asthma, and patient is allowed to reduce his steroid intake if asthma symptoms or incidence decrease (e.g., by 20%, 30% or 40% or more); oral Amphotericin B is maintained at same or reduced dosages until patient is able to cease or substantially reduce using oral steroid inhalants and/or has substantially no asthma or no detectable asthma. Over the next six months, patients are monitored for normal renal function and asthma parameters, including peak flow. In alternative embodiments, oral Amphotericin B is maintained until patients have a normal, or near normal (e.g., within 90% or 80% normal for age and sex) exhaled nitric oxide (NO) test (a standard test for the diagnosis and treatment of asthma).

In alternative embodiments, the orally formulated Amphotericin B is administered in conjunction with azithromycin and rifabutin, for about 8 to 12 weeks. The Amphotericin B, azithromycin and rifabutin can be given in separate pills, or as one formulation, or can be packaged together in a single manufacture such as a blister pack to ensure patient compliance with dosage regimen.

In alternative embodiments, the orally formulated Amphotericin B comprises one or more of:
- a FUNGILIN^{™} (Aspen Pharma Pty Ltd, New Zealand; iCo Therapeuics Inc., Vancouver, BC, Canada), optionally as tablets, suspensions or lozenges;
- a micronized formulation of Amphotericin B;
- a nanosuspension of Amphotericin B, optionally made by high-pressure homogenization, e.g., as described by Wasan et al, J. of Infectious Disease (2009) vol 200(3):357-360), or as described by Torrado et al Therapeutic Delivery (2012) vol 4(1):9-12, optionally the nanosuspension comprising Tween 80 (optionally at 0.5% w/w), Pluronic F68 (optionally at 0.25% w/w), and sodium cholate (optionally 0.05% w/w);
- a solubilized formulation of Amphotericin B, e.g., as described by Kravetz et al N. Engl. J. Med (1961) 265:183-184;
- Amphotericin B nanoparticles, optionally comprising PEGylated polylactic-polyglycolic acid copolymer (PLGA-PEG) nanoparticles (NPs) or equivalents, e.g., as described by Radwan et al, J. Drug Delivery (2017) vol 24(1):40-50;
- Amphotericin B attached to functionalized carbon nanotubes, e.g., as described in Torrado et al Therapeutic Delivery (2012) vol 4(1):9-12
- a lipid-based formulation of Amphotericin B comprising mono- and diglycerides with phospholipids; and/or
- an encochleated (a lipid-crystal nano-particle formulation) formulation of Amphotericin B, optionally MAT2203 (Matinas BioPharma Laboratories/ Matinas BioPharma Nanotechnologies, Inc., Bridgewater, NJ).

In alternative embodiment, patients have symptomatic refractory asthma with demonstrated airway hyper-responsiveness to hypertonic saline; where clinical features of severe refractory asthma include:
- A well-documented requirement for high dose inhaled corticosteroids (ICS), e.g., >= 880 mcg/day fluticasone propionate or equivalent daily, for at least 12 months;
- Using additional controller medication in addition to high dose ICS for at least 12 months;
- Persistent airflow obstruction indicated by a pre-bronchodilator FEV1<80% predicted at visit 1 or 2 or peak flow diurnal variability of >20% on 3 or more days during the run-in;
- History of 2 or more exacerbations requiring systemic corticosteroids in the previous 12 months;
- Evidence of asthma documented by airway reversibility, airway hyper-responsiveness or airflow variability - FEV1<FVC ratio < 70% and FEV1% predicted < 80%.

### Reference Example 2: Single-Patient Study of Asthma Treatment with Oral Amphotericin B

A 32-year-old male patient with quite marked asthma taking Salbutamol and steroid inhalations was invited to participate in a therapeutic study involving oral absorbable Amphotericin B. His original average of three readings Forced Expiratory Volume in one second (FEV1) was 200, and Forced Vital Capacity (FVC) was 370 with the FEV1:FVC ratio being 0.54. He stopped taking the Salbutamol and steroid inhalations, and was then commenced on oral Amphotericin B 200 mg given twice daily in an encapsulated format, administered simultaneously with oral rifabutin 150 mg and oral azithromycin 250 mg for 2 weeks. At the end of those 2 weeks, three readings were collected and averaged. The FEV1 was 320, the FVC was 390, and the FEV1:FVC ratio was 0.82. The increase from 200 to 320 of FEV1 was remarkable (60.0% increase), and the patient mentioned that he did not have to take any of his daily inhalant therapies for his asthma. He elected to remain on the combined treatment of oral Amphotericin B, rifabutin, and azithromycin for a further week (week 3).

### Example 3: Inhaled Amphotericin B to Treat Fungi

Because Amphotericin B (AMB) is not absorbed systemically during inhalation, it can be nebulized (generally in the Emergency Room (ER)) to reach clinically effective doses in the bronchi as assessed by bronchoscopic sampling. Delivery to the lungs can also be achieved on an ambulatory basis after discharge from hospital using an asthma 'puffer' or inhaler, generally used to administer agents such as salbutamol (Ventolin^{®}).

Parenterally, AMB can be quite toxic and, in particular, can cause renal damage. Inhaled AMB is mostly undetectable in blood after inhalation and is non-toxic, even after prolonged use. Some forms of inhaled AMB are retained in the tissues for many days after administration, thereby permitting infrequent inhalation dosing that can still treat tissues for fungal infection. There are several formulations of AMB that can be used for inhalation (see table below).

**Table. Formulations of amphotericin B (AMB includes all formulations)**

| Formulation | Brand name | Manufacturer | Carrier | Colloidal type | Size *(µm)* |
|---|---|---|---|---|---|
| AMB-DOC | Fungizone-R | Bristol Myers-Squibb | Desoxycholate | Micelle | 0.035 |
| L-AMB | AmBisome | Gilead Sciences | HSPC/DSPG/ Chol | Liposome | 0.08 |
| ABLC | Abelcet | The Liposome Company | DMPC/DMPG | Lipid ribbon | 1.6-11 |
| ABCD | Amphocil, Amphotec | Alza Corporation | Cholesteryl sulphate | Lipid disk | 0.11-0.12 |

| | | | | | |
|---|---|---|---|---|---|
| Acronyms Used: AMB, amphotericin B; HSPC, Hydrogenated Soy Phosphatldylcholine; DSPG, distearoylphosphatidylglycerol; Chol, cholesterol; DMPC, dimyristoylphosphatidylcholine; DMPG, dimyristoylphosphatidylglycerol. | | | | | |

In asthma and other indications, inhaled AMB products can be used either alone with administration weekly, x2/week, x3/week, x4/week, x5/week, or daily. In certain situations, e.g., severe disease, inhalations may exceed one per day, e.g., bid, tid, or even qds or more, in order to achieve initial high bronchial levels. Because AMB penetrates mucosa in the bronchi and alveoli, it can act specifically where fungi grow (deep within the bronchial walls). Hence, AMB could be administered as a single agent in an acute care setting such as the ER where it could be administered frequently through a nebuliser and then, as the patient improved, s/he could be discharged with a puffer device (inhaler) to maintain longer term inhalations that would be required less frequently. The dosage would vary from between 0.5mg per inhalation through to 500mg per inhalation.

The fungi that may be encountered in human lungs include, but are not limited to, *Candida albicans* (reported in 60% of the cases), followed by *Bipolaris* species (13%), *Schizophyllum commune* (11%), *Curvularia* species (8%), *Pseudallescheria boydii* species complex (3%) and, more rarely, *Alternaria alternata, Fusarium vasinfectum, Penicillium* species, *Cladosporium cladosporioides, Stemphylium languinosum, Rhizopus oryzae, Candida glabrata, Saccharomyces cerevisiae, Schizophyllum commune, Trichosporon beigelii, Aspergillus* species, or an *Aspergillus fumigatus,* an *Aspergillus flavus* or an *Aspergillus niger,* or *Scedosporium, Fusarium, Paecilomyces, Acremonium, Trichoderma and Cryptococcus gatti,* or *Histoplasma capsulatum, Coccidioides immitis, Blastomyces dermatitidis, Paracoccidioides brasiliensis, Sporothrix schenckii, Cryptococcus neoformans, Candida* species, *Mucor* species, *Pneumocystis* including *jiroveci, Blastomycosis,* and *Zygomycosis.*

Inhaled AMB may also be combined with other inhaled antifungal agents. Suitable inhaled antifungal agents include, but are not limited to, Abafungin, Allylamines, amorolfin, Bifonazole, butenafine, Candicidin, Filipin, Hamycin, naftifine, Natamycin, Nystatin, Rimocidin, terbinafine, Butoconazole, Clotrimazole, Econazole, Fenticonazole, Isoconazole, Ketoconazole, Luliconazole, Miconazole, Omoconazole, Oxiconazole, Sertaconazole, Sulconazole, Tioconazole, and triazoles (including, but not limited to, Albaconazole, Efinaconazole, Epoxiconazole, Fluconazole, Isavuconazole, Itraconazole, Posaconazole, Propiconazole, Ravuconazole, Terconazole, and Voriconazole).

Inhaled AMB may also be combined with oral antifungal agents, with the AMB being nebulised or inhaled in a puffer device or inhaler whilst the other antifungal agents, such as those listed above, are administered orally. Oral dosing, e.g. of itraconazole known to inhibit asthma [Denning et al (2009) m J Respir Crit Care Med 179:11-18] would boost efficacy of asthma treatment because intrapulmonary AMB would provide local efficacy combined with other asthma-effective oral circulating antifungal, in this case itraconazole. The nebuliser-inhaled AMB may also be combined with antibiotics as described above. Suitable antibiotics include, but are not limited to, Ansamycins (such as Rifampicin, Rifabutin or Rifalazil); Tetracyclines (such as doxycycline, minocycline, omadacycline, lymecycline or tetracycline hydrochloride); Lymecycline (although it is a tetracycline derivative it can be used in children above the age of 8); or Lefamulin, which is active against *Chlamydophila pneumoniae* and *Moraxella catarrhalis.*

**Table 1:**

| Antibiotic Classes | Generic name (Brand Name) |
|---|---|
| Aminoglycosides | Amikacin (Amikin), Gentamicin (Garamycin), Kanamycin (Kantrex), Neomycin (Neo-Fradin), Netilmicin (Netromycin), Tobramycin (Nebcin), Paromomycin (Humatin), Streptomycin (N/A), Spectinomycin (Trobicin) |
| Ansamycins | Geldanamycin (Trastuzumab), Herbimycin (N/A), Rifaximin (Xifaxan),Rifabutin(Mycobutin), Rifampicin (Rifampin), Rifalazil, Rifapentine; Tanespimycin |
| Carbacephem | Loracarbef (Lorabid) |
| Carbapenems | Ertapenem (Invanz), Doripenem (Doribax), Imipenem/Cilastatin (Primaxin), Meropenem (Merrem) |
| Cephalosporins (First generation) | Cefadroxil (Duricef), Cefazolin (Ancef), Cefalexin (Keflex) |
| Cephalosporins (Second generation) | Cefaclor (Distaclor), Cefprozil (Cefzil), Cefuroxime (Ceftin, Zinnat) |
| Cephalosporins (Third generation) | Cefixime (Cefspan), Cefdinir (Omnicef, Cefdiel), Cefditoren (Spectracef, Meiact), Cefoperazone (Cefobid), Cefotaxime (Claforan), Cefpodoxime (Vantin, Banadoz), Ceftazidime (Fortaz), Ceftibuten (Cedax), Ceftriaxone (Rocephin) |
| Cephalosporins (Fourth generation) | Cefepime (Maxipime) |
| Cephalosporins (Fifth generation) | Ceftaroline fosamil (Teflaro), Ceftobiprole (Zeftera) |
| Glycopeptides | Teicoplanin (Targocid), Vancomycin (Vancocin),Telavancin (Vibativ), Dalbavancin (Dalvance), Oritavancin (Orbactiv) |
| Lincosamides | Clindamycin (e.g., CLEOCIN^{™}, DALACIN^{™}, CLINACIN^{™}), Lincomycin (Lincocin) |
| Lipopeptide | Daptomycin (Cubicin) |
| Macrolides | Azithromycin (Zithromax, Sumamed, Xithrone), Clarithromycin (Biaxin), Erythromycin (Erythocin, Erythroped), Roxithromycin (N/A), Telithromycin (Ketek), Spiramycin (Rovamycine) |
| Monobactams | Aztreonam (Azactam) |
| Nitrofurans | Furazolidone (Furoxone), Nitrofurantoin (Macrodantin, Macrobid) |
| Nitroimidazoles | Tinidazole (Fasigyn, Simplotan, Tindamax), Metronidazole (Flagyl), Ornidazole (Ornigil), Secnidazole |
| Oxazolidinones | Linezolid (Zyvox), Posizolid (N/A), Radezolid (N/A), Torezolid (Sivextro); Cadazolid |
| Penicillins | Amoxicillin (Novamox, Amoxil), Ampicillin (Principen), Azlocillin, Dicloxacillin (Dynapen), Flucloxacillin (Floxapen), Mezlocillin (Mezlin), Methicillin (Staphcillin), Nafcillin (Unipen), Oxacillin (Prostaphlin), Penicillin G (Pentids), Penicillin V (Veetids), Piperacillin (Pipracil), Penicillin G (Pfizerpen), Temocillin (Negaban), Ticarcillin (Ticar) |
| Penicillin combinations | Amoxicillin/clavulanate (Augmentin), Ampicillin/sulbactam (Unasyn), Piperacillin/tazobactam (Zosyn), Ticarcillin/clavulanate (Timentin) |
| Polypeptides | Bacitracin (Baciguent), Colistin (Coly-Mycin-S), Polymyxin B |
| Quinolones/Fluoroq uinolones | Ciprofloxacin (Cipro, Ciproxin, Ciprobay), Enoxacin (Penetrex), Gatifloxacin (Tequin), Gemifloxacin (Factive), Levofloxacin (Levaquin), Lomefloxacin (Maxaquin), Moxifloxacin (Avelox), Nadifloxacin (Nadoxin), Nalidixic acid (NegGram), Norfloxacin (Noroxin), Ofloxacin (Floxin, Ocuflox), Trovafloxacin (Trovan), Grepafloxacin (Raxar), Sparfloxacin (Zagam), Temafloxacin (Omniflox) |
| Sulfonamides | Mafenide (Sulfamylon), Sulfacetamide (Sulamyd, Bleph-10), Sulfadiazine (Micro-Sulfon), Silver sulfadiazine (Silvadene), Sulfadimethoxine (Di-Methox, Albon), Sulfamethizole (Thiosulfil Forte), Sulfamethoxazole (Gantanol), Sulfanilimide (N/A), Sulfasalazine (Azulfidine), Sulfisoxazole (Gantrisin), Trimethoprim (Bactrim, Septra), Sulfamethoxazole (Gantanol), Sulfonamidochrysoidine (Prontosil) |
| Tetracyclines | Demeclocycline (Declomycin), Doxycycline (Vibramycin), Metacycline |
| | Minocycline (Minocin), Oxytetracycline (Terramycin), Tetracycline (Sumycin, Achromycin V, Steclin) |
| Drugs against mycobacteria | Clofazimine (Lamprene), Dapsone (Avlosulfon), Capreomycin (Capastat), Cycloserine (Seromycin), Ethambutol (Myambutol), Ethionamide (Trecator), Isoniazid (Nydrazid), Pyrazinamide (Aldinamide), Rifampicin (Rifadin, Rimactane), Rifabutin (Mycobutin), Rifapentine (Priftin), Streptomycin (N/A) |
| Others | Arsphenamine (Salvarsan), Chloramphenicol (Chloromycetin), Fosfomycin (Monurol, Monuril), Fusidic acid (N/A), Metronidazole (Flagyl), Mupirocin (Bactroban), Platensimycin (N/A), Quinupristin/Dalfopristin (Synercid), Thiamphenicol, Tigecycline (Tigacyl), Tinidazole (Tindamax Fasigyn), Trimethoprim (Proloprim, Trimpex) ; Fidaxomicin (Marocyclic antibiotic - Dificid); Ridinilazole; Ramoplanin; Nitazoxanide; Tizoxanide; Surotomycin; |

| | |
|---|---|
| N/A: Not available | |

The AMB may also be combined with any of the antibiotics disclosed herein, particularly agents such as nitroimidazoles and moxifloxacin.

In alternative embodiments, inhaled AMB may be combined with inhaled antibiotics, e.g., Gentamycin, Streptomycin, Vancomycin, Lincomycin, Penicillin, Metronidazole and others listed in Table 1.

Inhaled AMB (or equivalent antifungals) may be packed in an inhaler or puffer device for patient use, such as for daily inhalations of AMB (or equivalent antifungals) as a maintenance therapy. Inhaled AMB (or equivalent antifungals) for maintenance therapy may be co-prescribed with 2 or more antibiotics to treat any non-fungal component of the lung infection that may have precipitated the asthma attack. Suitable antibiotics for such use include, but are not limited to, any of those discussed above.

In alternative embodiments, the method of treatment may comprise, prior to AMB inhalations in ER, treatment with a biofilm-dissolving enzyme, such as Pulmozyme, followed by inhalation of the active antifungals and antibiotics as described above.

In alternative embodiments, combined Oral Anti-fungal agents may also be used with or without nebulised AMB. Suitable oral anti-fungal agents include, but are not limited to, those listed above.

The above treatments may be used in respiratory applications other than asthma, including, but not limited to, Cystic Fibrosis, Idiopathic Pulmonary fibrosis, COPD, Pneumonia, Chronic Bronchitis, and Sarcoidosis.

## Claims

1. A therapeutic combination of drugs comprising Amphotericin B, azithromycin, and rifabutin, wherein the Amphotericin B is formulated for administration by inhalation wherein the amphotericin B is formulated as a spray, an aerosol or a powder.

2. The therapeutic combination of drugs of claim 1, wherein the combination further comprises itraconazole.

3. The therapeutic combination of drugs of claim 1, wherein the combination further comprises gentamycin, or aztreonam.

4. The therapeutic combination of drugs of any preceding claim, wherein the amphotericin B is formulated for administration in an amount of about 250 mg per day, about 200 to 300 mg per day, about 300 to 500 mg per day, or about 100, 200, 250, 300, 350, 400 or 500 mg per day.

5. The therapeutic combination of drugs of any preceding claim for use in the treatment of an asthma, a bronchitis, a sinusitis, or rhinosinusitis.

6. The therapeutic combination of drugs for use of claim 5, for use in the treatment of an asthma.

7. The therapeutic combination of drugs for use of claim 5 or claim 6, wherein the treatment does not involve the cessation or reduction of use of emollients by the individual in need thereof.

8. The therapeutic combination of drugs for use of claim 5 or claim 6, wherein the treatment comprises cessation or reduction of use of emollients by the individual in need thereof.

9. The therapeutic combination of drugs for use of any one of claims 5-8, wherein the treatment further comprises use of or administration of one or more of a mucolytic agent, a steroid, a decongestant and a bronchodilator.

## Patentansprüche

1. Therapeutische Kombination aus Arzneimitteln, umfassend Amphotericin B, Azithromycin und Rifabutin, wobei das Amphotericin B zur Verabreichung durch Inhalation formuliert ist, wobei das Amphotericin B als ein Spray, ein Aerosol oder ein Pulver formuliert ist.

2. Therapeutische Kombination aus Arzneimitteln nach Anspruch 1, wobei die Kombination ferner Itraconazol umfasst.

3. Therapeutische Kombination aus Arzneimitteln nach Anspruch 1, wobei die Kombination ferner Gentamycin oder Aztreonam umfasst.

4. Therapeutische Kombination aus Arzneimitteln nach einem vorhergehenden Anspruch, wobei das Amphotericin B zur Verabreichung in einer Menge von etwa 250 mg pro Tag, etwa 200 bis 300 mg pro Tag, etwa 300 bis 500 mg pro Tag oder etwa 100, 200, 250, 300, 350, 400 oder 500 mg pro Tag formuliert ist.

5. Therapeutische Kombination aus Arzneimitteln nach einem vorhergehenden Anspruch zur Verwendung bei der Behandlung von Asthma, Bronchitis, Sinusitis oder Rhinosinusitis.

6. Therapeutische Kombination aus Arzneimitteln zur Verwendung nach Anspruch 5 zur Verwendung bei der Behandlung von Asthma.

7. Therapeutische Kombination aus Arzneimitteln zur Verwendung nach Anspruch 5 oder Anspruch 6, wobei die Behandlung nicht die Beendigung oder Reduzierung von Verwendung von Erweichungsmitteln durch das Individuum, das dessen bedarf, involviert.

8. Therapeutische Kombination aus Arzneimitteln zur Verwendung nach Anspruch 5 oder Anspruch 6, wobei die Behandlung Beendigung oder Reduzierung von Verwendung von Erweichungsmitteln durch das Individuum, das dessen bedarf, umfasst.

9. Therapeutische Kombination aus Arzneimitteln zur Verwendung nach einem der Ansprüche 5-8, wobei die Behandlung ferner Verwendung von oder Verabreichung von einem oder mehreren von einem mukolytischen Mittel, einem Steroid, einem Abschweller und einem Bronchodilatator umfasst.

## Revendications

1. Combinaison thérapeutique de médicaments comprenant de l'amphotéricine B, de l'azithromycine et de la rifabutine, dans laquelle l'amphotéricine B est formulée pour être administrée par inhalation, dans laquelle l'amphotéricine B est formulée sous forme d'un spray, d'un aérosol ou d'une poudre.

2. Combinaison thérapeutique de médicaments selon la revendication 1, dans laquelle la combinaison comprend en outre de l'itraconazole.

3. Combinaison thérapeutique de médicaments selon la revendication 1, dans laquelle la combinaison comprend en outre de la gentamicine ou de l'aztréonam.

4. Combinaison thérapeutique de médicaments selon une quelconque revendication précédente, dans laquelle l'amphotéricine B est formulée pour être administrée en une quantité d'environ 250 mg par jour, d'environ 200 à 300 mg par jour, d'environ 300 à 500 mg par jour, ou d'environ 100, 200, 250, 300, 350, 400 ou 500 mg par jour.

5. Combinaison thérapeutique de médicaments selon une quelconque revendication précédente, destinée à être utilisée dans le traitement d'un asthme, d'une bronchite, d'une sinusite ou d'une rhinosinusite.

6. Combinaison thérapeutique de médicaments destinée à être utilisée selon la revendication 5, destinée à être utilisée dans le traitement d'un asthme.

7. Combinaison thérapeutique de médicaments destinée à être utilisée selon la revendication 5 ou la revendication 6, dans laquelle le traitement n'implique pas l'arrêt ou la réduction de l'utilisation d'émollients par l'individu qui en a besoin.

8. Combinaison thérapeutique de médicaments destinée à être utilisée selon la revendication 5 ou la revendication 6, dans laquelle le traitement comprend l'arrêt ou la réduction de l'utilisation d'émollients par l'individu qui en a besoin.

9. Combinaison thérapeutique de médicaments destinée à être utilisée selon l'une quelconque des revendications 5 à 8, dans laquelle le traitement comprend en outre l'utilisation ou l'administration d'un ou plusieurs d'un agent mucolytique, d'un stéroïde, d'un décongestionnant et d'un bronchodilatateur.
